# FASCICULE DE BREVET EUROPEEN

(11) **EP 0 461 019 B1**
(45) Date de publication et mention de la délivrance du brevet: **25.10.1995**
(21) Numéro de dépôt: 91401446.9
(22) Date de dépôt: 04.06.1991
(51) Int. Cl.: A61F 2/32

(54) **Rotule pour prothèse**
Kugelgelenk für Prothese
Prosthesis ball joint

(30) Priorité: 06.06.1990 FR 9006978
(43) Date de publication de la demande: 11.12.1991
(73) Titulaire: Bouvet, Jean-Claude, F-91590 La Ferte Alais (FR)
(72) Inventeur: Bouvet, Jean-Claude, F-91590 La Ferte Alais (FR)
(74) Mandataire: Flavenot, Bernard

(56) Documents cités:
- FR-A- 2 597 747
- GB-A- 2 126 096
- US-A- 3 916 451
- US-A- 4 040 130
- US-A- 4 524 467

## Description

La présente invention concerne les rotules pour prothèses, telles que celles qui sont implantées dans le corps humain quand une articulation est détériorée, par exemple suite à une maladie ou à un accident, notamment celle de la hanche, du genou, etc.

Les chirurgiens sont très souvent amenés à pratiquer des opérations qui consistent à implanter des prothèses, dont les plus courantes sont celles de la hanche et du genou. En effet, suite à des accidents ou des maladies, beaucoup de personnes âgées, notamment, souffrent d'une détérioration de la tête du fémur qu'il est alors nécessaire de remplacer par des moyens comme une prothèse à rotule.

Cette rotule comporte une partie mâle généralement constituée par une portion de sphère montée par tous moyens sur une patte de fixation destinée à être implantée dans un os long, et, l'occurrence le fémur, et une partie femelle en forme de cupule complémentaire de la portion de sphère et de même rayon, apte a être implantée dans l'os du bassin, la partie mâle étant maintenue dans la partie femelle par tous moyens, notamment les tendons et les muscles du patient sur lequel est implantée la prothèse.

Dans le cas plus particulier de la hanche, la prothèse doit être conçue pour permettre les mêmes mouvements de la jambe par rapport au bassin que ceux qui sont normalement possible pour une articulation saine. C'est ainsi qu'une personne qui porte une telle prothèse doit pouvoir effectuer les mouvements de la marche dite "normale", par des oscillations de la jambe d'une amplitude ne dépassant généralement pas trente à quarante degrés par rapport à la verticale, que des mouvements plus forcés d'amplitudes pouvant atteindre cent vingt degrés, et même un peu plus.

Il est déjà connu une prothèse qui tente de répondre aux objectifs définis ci-dessus, celle qui est décrite dans le Brevet US-A-3 916 451. Ce document a trait à une rotule de prothèse dont la partie femelle comporte une coque ayant une première cavité de forme hémisphérique, et une cupule délimitée par deux surfaces hémisphériques extérieure et intérieure, la surface hémisphérique extérieure étant complémentaire de la surface hémisphérique de la première cavité et la surface hémisphérique intérieure définissait une seconde cavité ayant un rayon de courbure nettement inférieur au rayon de courbure de la première cavité, les centres de courbure des surfaces hémisphériques intérieure et extérieure de la cupule n'étant pas confondus. La cupule est disposée dans la première cavité de façon à pouvoir y pivoter et la seconde cavité est apte à recevoir en rotation une tête de forme sphérique, mais la disposition relative des deux centres de courbure ne permet pas une bonne stabilité des deux parties de la rotule. Ces deux parties peuvent alors, dans des mouvements de grande amplitude et sous des efforts même relativement faibles, arriver à se dissocier l'une de l'autre.

La présente invention a pour but de réaliser une rotule de prothèse trouvant une application particulièrement avantageuse, notamment, pour les prothèses de hanche, qui assure à la prothèse une parfaite stabilité et une durée de vie plus grande que celle des prothèses actuelles, tout en permettant des amplitudes de rotation très importantes.

Plus précisément, la présente invention a pour objet une rotule de prothèse comportant une partie mâle comprenant au moins une tête de forme sphérique apte à coopérer avec une partie femelle formant une cavité de forme hémisphérique complémentaire de la forme sphérique de la tête, ladite partie femelle comportant au moins une coque, ladite coque comportant une première cavité de forme sensiblement hémisphérique ayant une première valeur de rayon de courbure et étant réalisée dans un premier matériau, des moyens pour fixer solidairement ladite coque à un os support, une cupule délimitée par deux surfaces sensiblement hémisphériques respectivement extérieure et intérieure, la surface hémisphérique extérieure étant complémentaire de la surface hémisphérique de ladite première cavité et étant réalisée dans un deuxième matériau, la surface hémisphérique intérieure définissant une seconde cavité ayant une seconde valeur de rayon de courbure nettement inférieure à ladite première valeur de rayon de courbure et étant réalisée dans un troisième matériau, ladite cupule étant disposée dans ladite première cavité de façon à pouvoir y pivoter et ladite seconde cavité étant apte à recevoir en rotation ladite tête de forme sphérique réalisée dans un quatrième matériau, les centres de courbure des surfaces hémisphériques intérieure et extérieure de ladite cupule n'étant pas confondus, caractérisée par le fait que la distance séparant le centre de courbure de la surface hémisphérique intérieure, du pôle de la surface hémisphérique extérieure, est inférieure à la première valeur de rayon de courbure.

D'autres caractéristiques et avantages de la présente invention apparaîtront au cours de la description suivante donnée en regard des dessins annexés à titre illustratif, mais nullement limitatif, dans lesquels:
La figure 1 représente, vu en coupe et partiellement écorché, un mode de réalisation d'une rotule selon l'invention dans le cas d'une prothèse de hanche, et
La figure 2 représente une vue de face du mode de réalisation d'une rotule selon la figure 1 mettant en relief ses avantages.

Les deux figures représentant un même mode de réalisation d'une rotule de prothèse selon l'invention, les mêmes références y désignent les mêmes éléments, quelle que soit la figure sur laquelle ils apparaissent.

La rotule de prothèse illustrée comporte, comme toute rotule, une partie mâle 1 comprenant au moins une tête 2 de forme sphérique et une partie femelle 3 constituant une cavité de forme hémisphérique complémentaire de la forme sphérique de la tête, la tête 2 étant apte à être insérée dans cette partie femelle 3 et à coopérer avec elle par rotation.

Il est précisé que, dans cette description, le terme "hémisphérique" ne doit pas être uniquement compris dans sa signification stricte, c'est-à-dire qualifiant une forme ou une surface sphérique d'angle au centre strictement égal à cent quatre vingts degrés, mais également dans le sens "sensiblement hémisphérique" pour qualifier une forme sphérique d'angle au centre voisin, en plus ou en moins, de cette valeur.

La partie femelle 3 comporte au moins une coque 4 comprenant une première cavité 5 de forme hémisphérique ayant une première valeur de rayon de courbure, cette première cavité 5 étant réalisée dans un premier matériau dont la nature sera définie ci-après. A la coque 4 sont avantageusement associés des moyens 6 pour la fixer solidairement à un os support tel que, par exemple, l'os du bassin 7.

La partie femelle 3 comporte en outre une cupule 8 délimitée par deux surfaces hémisphériques, respectivement extérieure 9 et intérieure 10, la surface hémisphérique extérieure 9 étant complémentaire de la surface hémisphérique 11 de la première cavité 5, la surface hémisphérique intérieure 10 définissant une seconde cavité 12, cette seconde cavité ayant une seconde valeur de rayon de courbure nettement intérieure à la première valeur de rayon de courbure, la cupule 8 étant disposée dans la première cavité de façon à pouvoir y pivoter.

La surface hémisphérique extérieure 9 de la cupule 8 et la surface hémisphérique intérieure 10 sont respectivement réalisées dans un deuxième et un troisième matériaux. La nature de chacun de ces matériaux sera, elle aussi, définie ci-après.

Il est bien entendu que la seconde cavité 12 est apte à recevoir en rotation la tête 3 de forme sphérique réalisée dans un quatrième matériau.

Selon une caractéristique importante de la rotule, le deuxième matériau 14 a un coefficient de dureté supérieur à celui du premier matériau 13 et le quatrième matériau 16 a un coefficient de dureté supérieur à celui du troisième matériau 15.

Cette caractéristique de la structure de la rotule est l'une des caractéristiques lui conférant une durée de vie importante, en limitant l'usure de ses surfaces de frottement pendant son fonctionnement dans l'articulation qu'elle a permis de restaurer, par le fait que ce sont ainsi les surfaces les plus dures qui se déplacent sur les surfaces les moins dures.

Dans un mode de réalisation avantageux, les premier 13 et troisième 15 matériaux sont une matière plastique, par exemple du polyéthylène, le deuxième matériau 14 est un matériau métallique, par exemple de l'acier inoxydable, et le quatrième matériau 16 peut être un métal ou, comme cela est bien connu en lui-même, de la céramique.

Comme mentionné ci-avant la rotule comporte avantageusement des moyens 6 pour fixer solidairement la coque 4 à un os support. Dans le mode de réalisation illustré, ces moyens 6 comprennent notamment une enveloppe 20 définissant un logement 21 dans lequel la coque peut être enfichée et retenue par des moyens de rétention constitués, par exemple, par un clip 23 solidaire de la coque 4 et coopérant avec une percée 24 réalisée dans l'enveloppe 20.

Les moyens 6 comprennent en outre des moyens d'accrochage de l'enveloppe 20 avec l'os support 7, par exemple des lamages, comme illustré en 25 sur les deux figures, ou des ergots, des pattes, etc. Le lamage est une solution intéressante car il permet l'utilisation de vis 26 dont les têtes, en se logeant dans le creux des lamages, ne peuvent pas gêner l'enfichage de la coque 4 dans le logement 21 de l'enveloppe 20. De plus, de façon connue, la surface externe de cette enveloppe peut être avantageusement enduite d'un produit facilitant son accrochage avec l'os support par ostéogénèse, par exemple de l'hydroxyapatite ou analogue.

Une autre caractéristique importante de la rotule concerne la structure de la cupule 8. En effet, dans un mode avantageux de réalisation, la cupule 8 comprend une coupelle 30 réalisée dans le deuxième matériau 14 défini ci-avant, une chemise 31 enfichée dans la coupelle 30 et réalisée dans le troisième matériau 15, et des moyens de maintien 32 de la chemise 31 dans la coupelle 30. Ces moyens 32 sont par exemple constitués par un petit bourrelet 33 solidaire de la chemise 31 et enfiché dans une rainure 34 correspondante réalisée dans la paroi intérieure 35 de la coupelle 30.

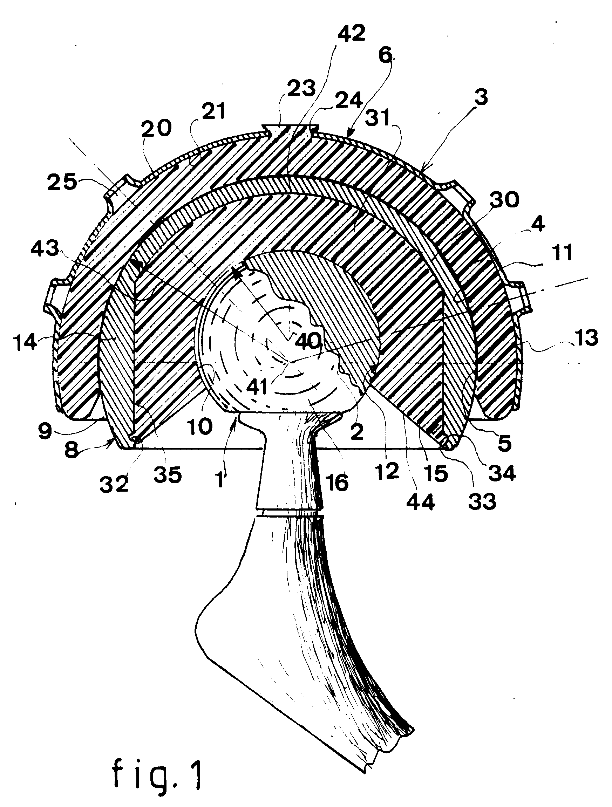

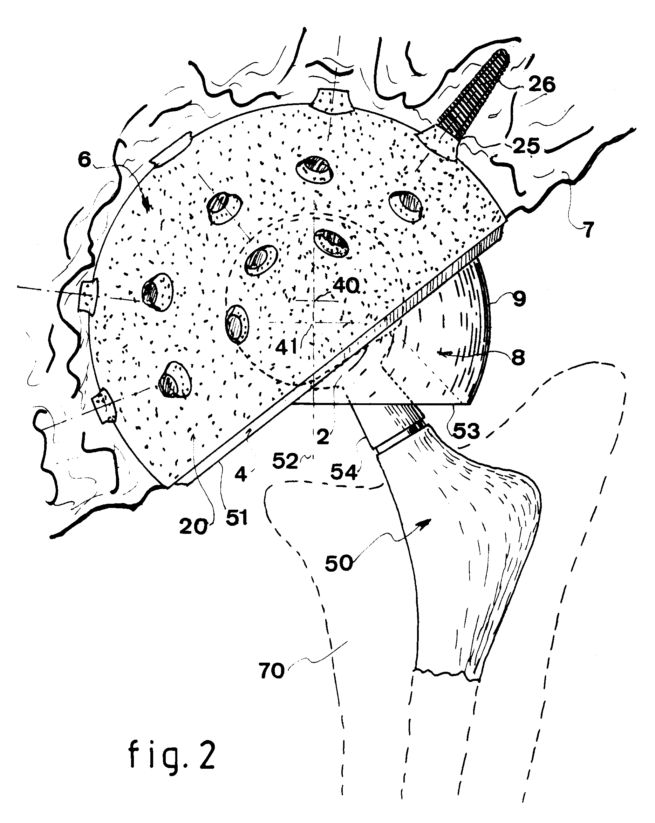

Dans le but de pouvoir positionner, comme illustré sur la figure 2, la cupule 8 dans la première cavité 5 et la tête sphérique 2 dans la cupule, les centres de courbure 40 et 41, respectivement des surfaces hémisphériques intérieure 10 et extérieure 9 de ladite cupule ne sont pas confondus. Ils sont même avantageusement définis de telle façon que la distance séparant la centre de courbure 40 de la surface hémisphérique intérieure, du pôle 42 de la surface hémisphérique extérieure, est inférieure à la première valeur de rayon de courbure défini par la distance référencée 43 sur la figure 1.

De plus, pour permettre les débattements angulaires du fémur 70 suffisants, comme mentionné ci-avant, pour assurer les mouvements d'une marche normale, c'est-à-dire les débattements d'une amplitude relativement faible, les deux surfaces hémisphériques extérieure 9 et intérieure 10 de la cupule 8 sont reliées par une surface sensiblement tronconique 44 dont le sommet est situé vers le fond de la seconde cavité 12, cette surface sensiblement tronconique constituant l'ouverture d'entrée de cette seconde cavité, par laquelle la tête sphérique 2 est mise en place dans la partie femelle 3 de la rotule.

La rotule de prothèse décrite ci-dessus trouve une application particulièrement avantageuse dans la réalisation d'une prothèse de hanche, comme illustré sur la figure 2. Dans ces conditions, la coque 4 est implantée dans l'os du bassin 7 par l'intermédiaire des moyens 6 et la tête 2 est solidarisée au fémur au moyen d'une patte de fixation 50 enfichée dans le fémur de façon connue en elle-même, l'implantation de la coque étant effectuée de façon que, lorsque le patient est dans la position debout, la face d'entrée 51 de la coque soit inclinée sur la verticale d'un angle de l'ordre de quarante cinq degrés.

Dans cette position, les efforts transmis à la tête ont une composante verticale très importante et, du fait que les deux centres de courbure 40 et 41 ne sont pas confondus et qu'ils sont situés l'un par rapport à l'autre comme défini ci-dessus, la cupule 8 qui enferme la tête 2 a alors tendance à se positionner dans la première cavité 5 de façon que les deux centres de courbures 40 et 41 soient sur une même verticale 52, c'est-à-dire avec son ouverture 53 située sensiblement dans un plan horizontal.

Lorsque le patient se déplace avec des mouvements de marche normale, son fémur 70 subit des oscillations qui ne dépassent généralement pas une valeur angulaire d'environ trente degrés de part et d'autre de la verticale. Dans ces conditions, seule la tête 2 de la prothèse subit des rotations par rapport à la cupule 8, cette dernière restant sensiblement immobile par rapport à la coque 4.

Par contre, lorsque le patient portant une telle prothèse est obligé d'effectuer des mouvements de plus grande amplitude, le col 54 de la patte 50 vient butter contre la surface tronconique 44 définie ci-avant et entraîne la rotation de la cupule 8 dans la cavité 5 de la coque. Cependant, ces mouvements forcés de grande amplitude sont relativement rares, surtout pour les personnes sur lesquelles sont implantées de telles prothèses. Il s'en suit que les deux surfaces de frottement de la rotule les plus grandes, c'est-à-dire la surface de la première cavité 5 et la surface hémisphérique extérieure 9 de la cupule 8, n'entrent en friction que rarement, ce qui permet, en pratique, de limiter l'usure de la rotule à celle des deux autres surfaces de frottement plus petites, la surface hémisphérique intérieure 10 et celle de la tête 2.

La structure de la rotule selon l'invention lui confère une durée de vie plus importante que celle des rotules de l'art antérieur par réduction de l'usure des surfaces de friction, tout en permettant les mêmes amplitudes d'oscillations.

## Revendications

1. Rotule de prothèse comportant une partie mâle (1) comprenant au moins une tête (2) de forme sphérique apte à coopérer avec une partie femelle (3) formant une cavité de forme hémisphérique complémentaire de la forme sphérique de la tête, ladite partie femelle comportant au moins:
une coque (4), ladite coque comportant une première cavité (5) de forme sensiblement hémisphérique ayant une première valeur de rayon de courbure et étant réalisée dans un premier matériau (13),
des moyens (6) pour fixer solidairement ladite coque à un os support (7),
une cupule (8) délimitée par deux surfaces sensiblement hémisphériques extérieure (9) et intérieure (10), la surface hémisphérique extérieure étant complémentaire de la surface hémisphérique de ladite première cavité et étant réalisée dans un deuxième matériau (14), la surface hémisphérique intérieure définissant une seconde cavité (12) ayant une seconde valeur de rayon de courbure nettement inférieure à ladite première valeur (43) de rayon de courbure et étant réalisée dans un troisième matériau (15), ladite cupule étant disposée dans ladite première cavité (5) de façon à pouvoir y pivoter et ladite seconde cavité étant apte à recevoir en rotation ladite tête (2) de forme sphérique réalisée dans un quatrième matériau (16), les centres de courbure des surfaces hémisphériques intérieure (10) et extérieure (9) de ladite cupule n'étant pas confondus,
caractérisée par le fait que la distance séparant le centre de courbure (40) de la surface hémisphérique intérieure (10), du pôle (42) de la surface hémisphérique extérieure (9), est inférieure à la première valeur (43) de rayon de courbure.

2. Rotule selon la revendication 1, caractérisée par le fait que ledit deuxième matériau (14) a un coefficient de dureté supérieur à celui du premier matériau (13) et que ledit quatrième matériau (16) a un coefficient de dureté supérieur à celui du troisième matériau (15).

3. Rotule selon l'une des revendications 1 à 2, caractérisée par le fait que lesdits moyens (6) pour fixer solidairement ladite coque à un os support comportent une enveloppe (20) définissant un logement (21), ladite coque étant enfichée dans ledit logement, des moyens de rétention de ladite coque dans ladite enveloppe, et des moyens d'accrochage de ladite enveloppe avec ledit os support (7).

4. Rotule selon la revendication 3, caractérisée par le fait que les moyens de rétention de ladite coque (4) dans ladite enveloppe (20) sont constitués par un clip (23) solidaire de ladite coque et coopérant avec une percée (24) réalisée dans ladite enveloppe.

5. Rotule selon la revendication 3, caractérisée par le fait que les moyens d'accrochage de ladite enveloppe avec ledit os support comportent au moins l'un des éléments suivants: un lamage (25), un ergot, une patte.

6. Rotule selon l'une des revendications 1 à 5, caractérisée par le fait que ladite cupule (8) comporte une coupelle (30) réalisée dans ledit deuxième matériau, une chemise (31) enfichée dans ladite coupelle et réalisée dans ledit troisième matériau (15), et des moyens de maintien (32) de ladite chemise dans ladite coupelle.

7. Rotule selon l'une des revendications 1 à 6, caractérisée par le fait que lesdits premier (13) et troisième (15) matériaux sont une matière plastique et que le deuxième (14) est un matériau métallique.

8. Rotule selon l'une des revendications 1 à 7, caractérisée par le fait que les deux dites surfaces hémisphériques extérieure (9) et intérieure (10) de ladite cupule (8) sont reliées par une surface sensiblement tronconique (44) dont le sommet est situé vers le fond de ladite seconde cavité (12), ladite surface sensiblement tronconique constituant l'ouverture d'entrée (53) de ladite seconde cavité.

## Claims

1. A prosthesis ball joint including a male portion (1) comprising at least one spherically-shaped head (2) suitable for co-operating with a female portion (3) forming a cavity of hemispherical shape complementary to the spherical shape of the head, said female portion including at least:
a shell (4), said shell including a first cavity (5) of substantially hemispherical shape having a radius of curvature of a first value and being made of a first material (13);
means (6) for fixing said shell securely to a supporting bone (7); and
a cup assembly (8) defined by two substantially hemispherical surfaces comprising an outer surface (9) and an inner surface (10), the outer hemispherical surface being complementary to the hemispherical surface of said first cavity and being made of a second material (14), the inner hemispherical surface defining a second cavity (12) having a radius of curvature of a second value that is considerably smaller than said first valve (43) for a radius of curvature, and being made of a third material (15), said cup assembly being disposed in said first cavity (5) in such a manner as to be capable of pivoting therein, and said second cavity being suitable for rotatably receiving said head (2) of spherical shape which is made out of a fourth material (16), the centers of curvature of the inner and outer hemispherical surfaces (10, 9) of said cup assembly not coinciding,
characterized by the fact that the distance between the center of curvature (40) of the inner hemispherical surface (10) and the pole (42) of the outer hemispherical surface (9) is less than the first value (43) for a radius of curvature.

2. A ball joint according to claim 1, characterized by the fact that said second material (14) has a hardness coefficient greater than that of the first material (13), and that said fourth material (16) has a hardness coefficient greater than that of the third material (15).

3. A ball joint according to claim 1 or 2, characterized by the fact that said means (6) for securely fixing said shell to a supporting bone comprise a case (20) defining a housing (21), said shell being engaged in said case, means for retaining said shell in said case, and means for securing said case to said supporting bone (7).

4. A ball joint according to claim 3, characterized by the fact that the means for retaining said shell (4) in said case (20) are constituted by a clip (23) secured to said shell and co-operating with a hole (24) formed in said case.

5. A ball joint according to claim 3, characterized by the fact that the means for securing said case to said supporting bone include at least one of the following elements: a punched countersink (25), a lug, a tab.

6. A ball joint according to any one of claims 1 to 5, characterized by the fact that said cup assembly (8) includes a cup (30) made of said second material, a lining (31) engaged in said cup and made of said third material (15), and means (32) for holding said lining in said cup.

7. A ball joint according to any one of claims 1 to 6, characterized by the fact that said first and third materials (13, 15) are a plastics material and said second material (14) is a metal material.

8. A ball joint according to any one of claims 1 to 7, characterized by the fact that said outer and inner hemispherical surfaces (9, 10) of said cup assembly (8) are interconnected by a substantially frustoconical surface (44) whose apex is situated towards the bottom of said second cavity (12), said substantially frustoconical surface constituting the inlet opening (53) of said second cavity.

## Patentansprüche

1. Prothesenkugelgelenk, umfaßend eine Schaftpartie (1) mit mindestens einem Kopf (2) sphärischer Form, ausgebildet zum Zusammenwirken mit einer Sitzpartie (3), die einen Hohlraum halbkugeliger Form komplementär zur sphärischen Form des Kopfes bildet, welche Sitzpartie mindestens umfaßt:
Eine Schale (4), die einen ersten Hohlraum (5) im wesentlichen halbkugeliger Form aufweist mit einem ersten Krümmungsradiuswert und ausgebildet aus einem ersten Material (13),
Mittel (6) zum starren Befestigen der Schale an einem Stützknochen (7),
einen Napf (8) begrenzt von im wesentlichen halbkugeligen äußeren (9) bzw. inneren (10) Oberflächen, wobei die äußere halbkugelige Oberfläche komplementär zur halbkugeligen Oberfläche des ersten Hohlraums ist und aus einem zweiten Material (14) hergestellt ist, während die halbkugelige innere Oberfläche einen zweiten Hohlraum (12) begrenzt mit einem zweiten Krümmungsradiuswert, der deutlich kleiner ist als der erste Wert (43) des Krümmungsradius und hergestellt ist aus einem dritten Material (15), welcher Napf in den ersten Hohlraum (5) derart angeordnet ist, daß er darin kippbeweglich ist und der zweite Hohlraum ausgebildet ist zur drehbeweglichen Aufnahme des Kopfes (2) kugeliger Form, der aus einem vierten Material (16) hergestellt ist, wobei die Krümmungszentren der inneren (10) bzw. äußeren (9) halbkugeligen Oberflächen des Napfes nicht zusammenfallen, **dadurch gekennzeichnet**, daß die Distanz zwischen dem Krümmungszentrum (40) der inneren halbkugeligen Oberfläche (10) von dem Pol (42) der äußeren halbkugeligen Oberfläche (9) kleiner ist als der erste Wert (43) des Krümmungsradius.

2. Kugelgelenk nach Anspruch 1, dadurch gekennzeichnet, daß das zweite Material (14) einen Härtekoeffizienten aufweist, der größer ist als der des ersten Materials (13) und daß das vierte Material (16) einen Härtekoeffizienten aufweist, der größer ist als der des dritten Materials (15).

3. Kugelgelenk nach einem der Ansprüche 1 bis 2, dadurch gekennzeichnet, daß die Mittel (6) zum starren Befestigen der Schale an einem Stützknochen eine eine Aufnahme (21) begrenzende Hülle (20) umfassen, wobei die Schale in die Aufnahme eingefügt ist, Haltemittel der Schale in der Hülle und Mittel zum Verankern der Hülle an dem Stützknochen (7).

4. Kugelgelenk nach Anspruch 3, dadurch gekennzeichnet, daß die Haltemittel der Schale (4) in der Hülle (20) von einem Clip (23) gebildet sind, der mit der Schale verbunden ist und mit einer in der Hülle vorgesehenen Durchbrechung (24) zusammenwirkt.

5. Kugelgelenk nach Anspruch 3, dadurch gekennzeichnet, daß die Mittel zum Verankern der Hülle an dem Stützknochen mindestens eins der folgenden Elemente umfassen: Eine Senkung (25), einen Fortsatz, eine Kralle.

6. Kugelgelenk nach einem der Ansprüche 1 bis 5, dadurch gekennzeichnet, daß der Napf (8) einen aus dem zweiten Material hergestellten Topf (30) umfaßt, in den eine Auskleidung (31) eingefügt ist, die aus dem dritten Material (15) hergestellt ist sowie Haltemittel (32) der Auskleidung in dem Topf.

7. Kugelgelenk nach einem der Ansprüche 1 bis 6, dadurch gekennzeichnet, daß das erste (13) und dritte (15) Material ein Kunststoffmaterial sind und daß das zweite (14) ein metallisches Material ist.

8. Kugelgelenk nach einem der Ansprüche 1 bis 7, dadurch gekennzeichnet, daß die beiden genannten äußeren (9) bzw. inneren (10) halbkugeligen Oberflächen des Napfes (8) miteinander über eine im wesentlichen kegelstumpfförmige Oberfläche (44) verbunden sind, deren Spitze in Richtung des Bodens des zweiten Hohlraums (12) liegt, welche im wesentlichen kegelstumpfförmige Oberfläche die Einfügeöffnung (53) des zweiten Hohlraums bildet.
